(19) Europäisches Patentamt
European Patent Office
Office européen des brevets

(11) Veröffentlichungsnummer: **0 010 740**
**B1**

(12) # EUROPÄISCHE PATENTSCHRIFT

(45) Veröffentlichungstag der Patentschrift: **21.04.82**

(51) Int. Cl.³: **C 07 D 493/10**, C 07 D 491/20, C 07 D 491/22, B 41 M 5/16

(21) Anmeldenummer: **79104148.6**

(22) Anmeldetag: **26.10.79**

(54) **Spirodipyrane und ihre Verwendung als Farbbildner für Kopierverfahren.**

(30) Priorität: **03.11.78 DE 2847690**

(43) Veröffentlichungstag der Anmeldung: **14.05.80 Patentblatt 80/10**

(45) Bekanntmachung des Hinweises auf die Patenterteilung: **21.04.82 Patentblatt 82/16**

(84) Benannte Vertragsstaaten: **BE CH DE FR GB IT**

(56) Entgegenhaltungen:
**DE-A-2 703 811**
**FR-A-2 190 816**
**FR-A-2 228 775**
**FR-A-2 344 559**

(73) Patentinhaber: **BASF Aktiengesellschaft, Carl-Bosch-Strasse 38, D-6700 Ludwigshafen (DE)**

(72) Erfinder: **Oberlinner, Andreas, Dr., Bruesseler Ring 53, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Spirodipyrane und ihre Verwendung als Farbbildner für Kopierverfahren

Die Erfindung betrifft Farbbildner der allgemeinen Formel I

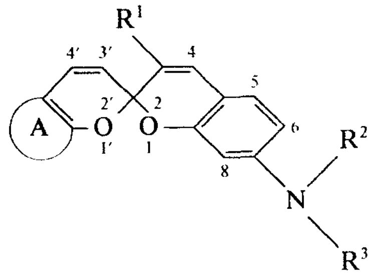

(I)

in der

A den Rest eines ankondensierten Benzolringes, der gegebenenfalls durch $C_1$- bis $C_4$-Alkyl, $C_1$- bis $C_4$-Alkoxy, Nitro, Chlor, Brom oder $C_1$- bis $C_6$-Alkoxycarbonyl substituiert ist, oder eines in 2,1-Stellung ankondensierten Naphthalinringes, der gegebenenfalls durch Chlor, Brom oder $C_1$- bis $C_6$-Alkoxycarbonyl substituiert ist,

$R^1$ $C_1$- bis $C_{16}$-Alkyl, Phenyl, durch $C_1$- bis $C_4$-Alkyl, $C_1$- bis $C_4$-Alkoxy, Chlor oder Brom substituiertes Phenyl, oder $C_2$- bis $C_{10}$-Phenylalkyl und

$R^2$ und $R^3$ unabhängig voneinander Wasserstoff, $C_1$- bis $C_{12}$-Alkyl, durch Cyan, Chlor, Methoxy oder Äthoxy substituiertes $C_2$- bis $C_4$-Alkyl, $C_5$- bis $C_7$-Cycloalkyl, $C_7$- bis $C_{10}$-Phenylalkyl oder ein gegebenenfalls durch 1 bis 3 Methyl substituiertes und an das in 6- und/oder 8-Stellung befindliche C-Atom des Benzringes gebundenes Trimethylen, oder

$R^2$ $C_1$- bis $C_4$-Alkyl und

$R^3$ Phenyl, durch Chlor, Brom, $C_1$- bis $C_3$-Alkyl, Methoxy oder Äthoxy substituiertes Phenyl oder

$R^2$ Benzyl und

$R^3$ $\beta$-Cyanäthyl oder die Gruppe

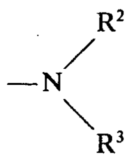

Morpholinyl, das gegebenenfalls durch ein oder zwei Methyl substituiert ist, Pyrrolidinyl, N'-$C_1$- bis $C_4$-Alkyl-piperazinyl oder Isoindolinyl bedeuten.

Die Spirodipyrane der Formel I sind schwach farbige bis farblose Verbindungen, deren Lösungen in einem inerten, organischen Lösungsmittel in Kontakt mit elektronenanziehenden Substanzen Färbungen in violetten, blauen bis grünen Farbtönen geben. Typische Beispiele für Elektronenaceptorsubstanzen sind Carbon- oder Mineralsäuren, Kaolin, Bentonit, aktivierter Ton, Aluminiumsilikat, Attapulgit oder jeder beliebige Ton, sauer reagierende polymere Materialien, wie Kondensationsprodukte auf der Basis von Phenolen und/oder Phenolsulfonsäuren, ferner Metalloxide oder -salze, wie Zinkoxid, Aluminiumoxid, Zinkchlorid, Eisenstearat oder Cobaltnaphthenat.

Aufgrund dieser Eigenschaften sind die neuen Verbindungen der Formel I als Farbbildner für druckempfindliche Aufzeichnungsmaterialien oder Kopiermaterialien geeignet.

Die erfindungsgemäßen Verbindungen der Formel I haben gegenüber den in der DE-PS 2 232 364 beschriebenen strukturisomeren Verbindungen mit einem Substituenten in 3'-Stellung in druckempfindlichen Durchschreibsystemen den Vorteil, daß die neuen Farbbildner auf nicht beschichtetem Streichrohpapier praktisch keine Tendenz zur Farbbildung zeigen. Beim Durchschreiben entsteht daher auf der Rückseite des mit dem Farbbildner beschichteten Deckblattes keine Spiegelschrift. Aus dem gleichen Grund erfolgt bei ungewollter Zerstörung der Kapseln keine Verschmutzung (Anfärbung) der Seite des Blattes, welche die Mikrokapselschicht trägt.

Aufgrund ihres Absorptionsverhaltens sind die erfindungsgemäßen Verbindungen zusammen mit weiteren gelben bis roten Farbbildnern gut für die Herstellung von schwarz durchschreibenden Farbbildnermischungen geeignet.

Für die Anwendung in druckempfindlichen Kopiersystemen ist es vorteilhaft, die erfindungsgemäßen Verbindungen in bekannter Weise in organischen Lösungsmitteln wie Chlorparaffinen, halogeniertem oder teilhydriertem Diphenyl, Alkylbenzol, Alkylnaphthalin, alkyliertem Dibenzylbenzol, Paraffinöl, Mineralöl oder auch in üblichen Lösungsmitteln wie Toluol, Xylol, in Form einer Lösung oder Suspension in Mikrokapseln einzuschließen und damit die Papieroberfläche zu beschichten. In

Kontakt mit elektronenanziehenden Materialien entsteht dann bei entsprechendem Schreib- oder Typendruck ein Schriftbild in violetter bis blauer bis grüner Farbe.

Geeignete Verfahren zur Herstellung von Mikrokapseln sind z. B. in den US-PS 2 800 457 und 2 800 458 und in der DE-PS 2 119 933 beschrieben.

Da die erfindungsgemäßen Verbindungen (I) auch in wäßriger Suspension stabiler sind als die Farbbildner des Standes der Technik, erhält man mit ersteren praktisch farblose Mikrokapseldispersionen.

Man kann die erfindungsgemäßen Verbindungen der allgemeinen Formel I auch nach dem in der US-PS 3 103 404 beschriebenen Verfahren in Wachs oder Öl-Wachs-Mischungen fein verteilen und mit diesen Mischungen Träger, wie Folien oder Papier, beschichten. Man erhält druckempfindliche Materialien, die zum Durchschreiben auf mit Elektronenakzeptorsubstanzen beschichteten Papieren geeignet sind und die nach Gebrauch wie Kohlepapier entfernt werden.

Für die Verwendung als Farbbildner sind aus anwendungstechnischen Gründen Spirodipyrane der Formel

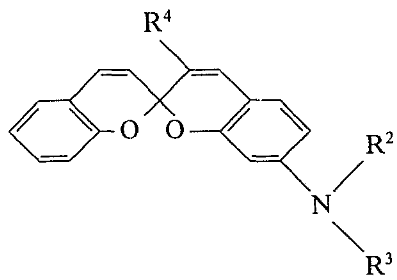

(Ia)

bevorzugt, in der $R^4$ $C_1$- bis $C_4$-Alkyl ist und $R^2$ und $R^3$ die vorstehend angegebenen Bedeutungen haben.

Von der Anwendung für druckempfindliche Kopierverfahren her gesehen sind die folgenden Verbindungen von besonderer Bedeutung:

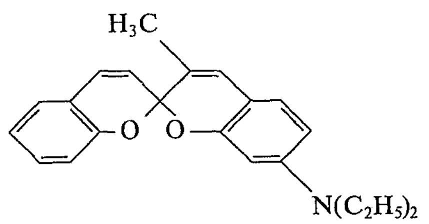

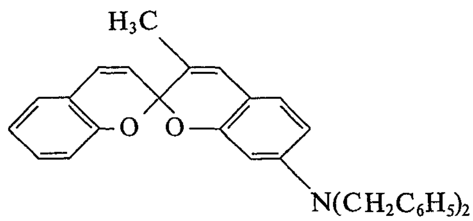

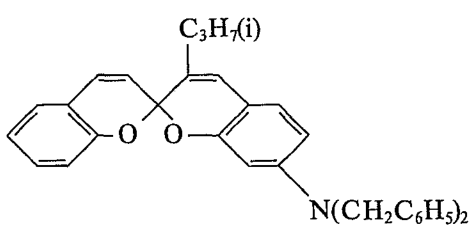

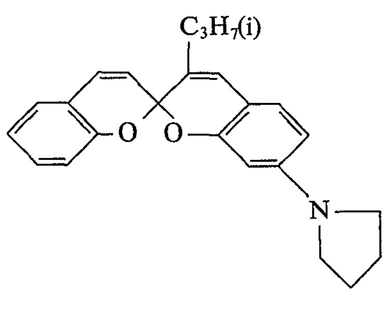

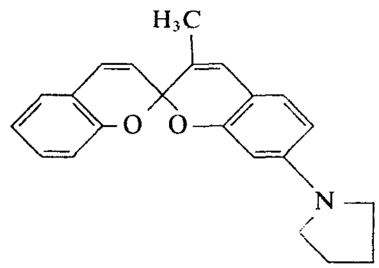

Die Synthese der Farbbildner erfolgt nach dem folgenden Reaktionsschema in an sich bekannter Weise durch Cyclisierung der Benzopyryliumverbindungen der Formel IV. Letztere werden z. B. durch Kondensation von o-Hydroxy-styrylverbindungen der Formel II mit N-substituierten p-Aminosalicylaldehyden der Formel III erhalten. Die Verbindungen der Formel IV können auch durch Umsetzung der Benzopyryliumsalze der Formel VI mit Aldehyden der Formel V nach dem folgenden Schema in an sich bekannter Weise hergestellt werden:

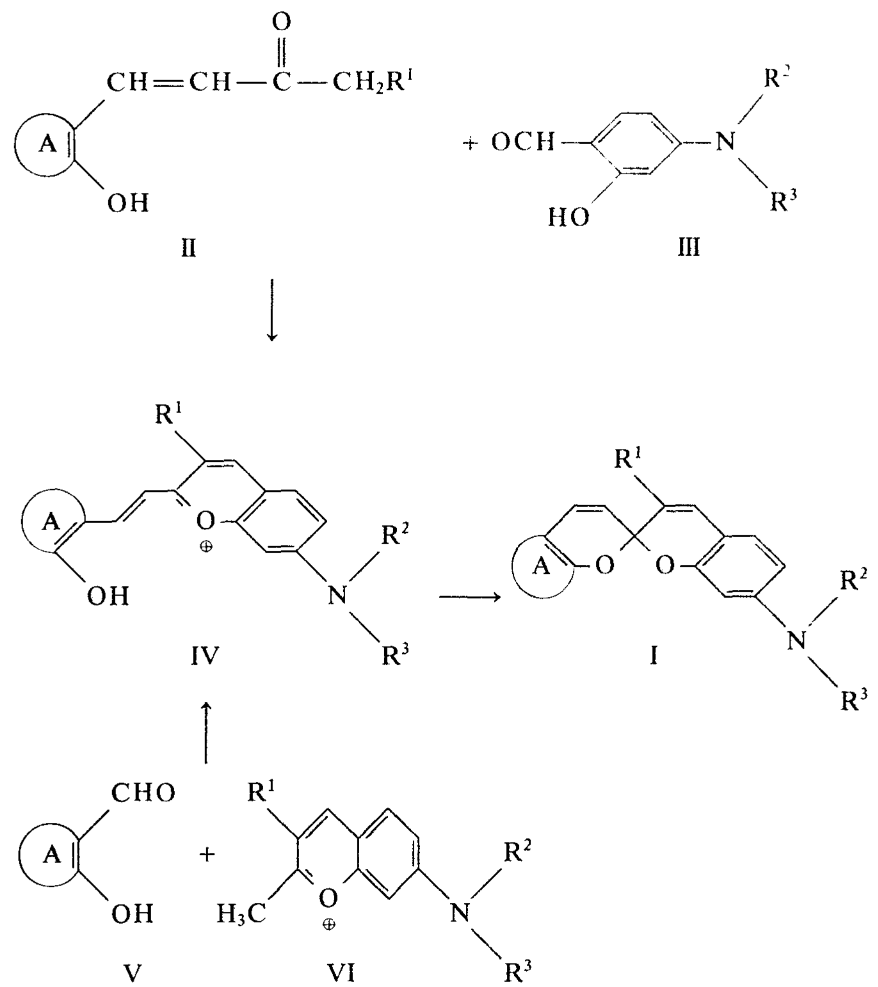

II III

IV I

V VI

Die Kondensation erfolgt zweckmäßigerweise in inerten organischen Lösungsmitteln, wie Alkoholen, Carbonsäuren, Carbonsäureanhydriden, Carbonsäureamiden, Kohlenwasserstoffen oder Acetonitril, gegebenenfalls in Gegenwart saurer oder basischer Kondensationsmittel, wie Zinkchlorid, Phosphorsäure, Chlorwasserstoff, Toluolsulfonsäure, Borsäure, Pyridin, Triäthylamin, Ammoniumacetat unter üblichen Kondensationsbedingungen.

In der Regel wird die Kondensation bei Temperaturen im Bereich von 20 bis 120° C ausgeführt.

Der Ringschluß zum Pyranderivat kann zusammen mit der Kondensation oder anschließend an diese im selben oder in einem getrennten Arbeitsgang, gegebenenfalls in Gegenwart von Basen, wie Natrium- oder Kaliumhydroxid, Natrium- oder Kaliumcarbonat, Natrium- oder Kaliumacetat, Ammoniak, aliphatischen Aminen, Pyridin, in üblicher Weise durchgeführt werden. Die aus dieser Lösung sich abscheidenden kristallisierten Spirodipyranverbindungen sind dann direkt oder nach

einer Reinigung z. B. durch Umkristallisieren oder Umfällen als Farbbildner für Kopierverfahren verwendbar.

Für die Herstellung der Verbindung (IV) kommen als Ausgangsverbindungen solche der Formeln II und III oder V und VI z. B. in Betracht:

a) 2-Oxy-styryl-ketone der Formel II:
Äthyl-(2-oxystyryl)-keton,
n-Propyl-(2-oxy-styryl)-keton,
Isobutyl-(2-oxy-styryl)-keton,
Äthyl-(2-oxy-5-chlor-styryl)-keton,
Äthyl-(2-oxy-5-brom-styryl)-keton,
Isopentyl-(2-oxy-styryl)-keton,
Isooctyl-(2-oxy-styryl)-keton,
Isohexyl-(2-oxy-styryl)-keton;

b) Aldehyde der Formel III:
4-N,N-Dimethylaminosalicylaldehyd,
4-N,N-Diäthylaminosalicylaldehyd,
4-N,N-Di-n-propylaminosalicylaldehyd,
4-N,N-Didodecylaminosalicylaldehyd,
4-N,N-Dibenzylaminosalicylaldehyd,
4-N,N-Diphenäthylaminosalicylaldehyd,
4-N-Methyl-N-p-tolyl-aminosalicylaldehyd,
4-N-Methyl-N-p-chlorphenyl-aminosalicylaldehyd,
4-N-Pyrrolidyl-salicylaldehyd,
4-N-Piperidinyl-salicylaldehyd,
4-N-Morpholinyl-salicylaldehyd,
4-N,N-Di-$\beta$-chloräthylamino-salicylaldehyd,
4-N-$\beta$-Methoxyäthyl-N-methylamino-salicylaldehyd,
4-N-Cyclohexyl-N-methylamino-salicylaldehyd,
4-N,N-Di-$\beta$-cyanäthylamino-salicylaldehyd,
4-N-Isoindolinyl-salicylaldehyd,
4-N-Methyl-N-(p-methoxyphenyl)-amino-salicylaldehyd
sowie die Verbindungen der Formel

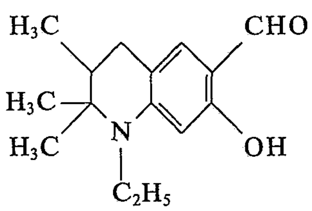

und

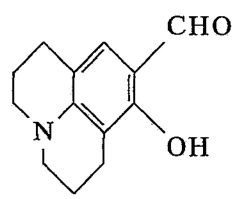

c) Aldehyde der Formel V:
Salicylaldehyd,
2-Oxy-1-naphthaldehyd,
6-Chlor-2-oxy-1-naphthaldehyd,
6-Brom-2-oxy-1-naphthaldehyd,
8-Methoxycarbonyl-2-oxy-1-naphthaldehyd,
8-Äthoxycarbonyl-2-oxy-1-naphthaldehyd,
4-Methoxysalicylaldehyd,
6-Methoxycarbonyl-salicylaldehyd,
5-tert.-Butyl-salicylaldehyd,
5-Nitro-salicylaldehyd;

d) Benzoypyryliumsalze der Formel VI in Form ihrer Chloride, Perchlorate, Tetrafluoroborate, Tetrachloroferrate, Trichlorzinkate:
2-Methyl-3-phenyl-7-dimethylamino-benzopyryliumsalz,
2-Methyl-3-p-tolyl-7-dimethylamino-benzopyryliumsalz,
2-Methyl-3-p-chlorphenyl-7-dimethylamino-benzopyryliumsalz,
2-Methyl-3-p-methoxyphenyl-7-dimethylamino-benzopyryliumsalz,
2-Methyl-3-phenyl-7-N-pyrrolidinyl-benzopyryliumsalz.

Die Herstellung und Isolierung der neuen Spirodipyranverbindung wird durch die folgenden Beispiele weiter erläutert.

Beispiel 1

In 80 Teilen Methanol werden 20 Teile Isobutyl-(2-oxy-styryl)-keton, 14 Teile Zinkchlorid und 19 Teile 4-Diäthylaminosalicylaldehyd gelöst. Man leitet in die Lösung bei 40 bis 50°C bis zur Sättigung Chlorwasserstoff ein und rührt 12 Stunden bei Raumtemperatur nach. Der kristalline Farbstoff wird aus dem abgekühlten Reaktionsgemisch isoliert und in 150 Teilen 25%iger Ammoniaklösung und 250 Teilen Toluol bis zur vollständigen Aufhellung gerührt. Die Toluolphase wird abgetrennt, mit Natriumsulfat getrocknet und auf ein Drittel ihres ursprünglichen Volumens eingeengt. Man gibt 100 Teile Methanol zu und fällt aus dieser Lösung 20 Teile 3-Isopropyl-7-diäthylamino-2,2′-spirodi-(2H-1-benzopyran) der Formel

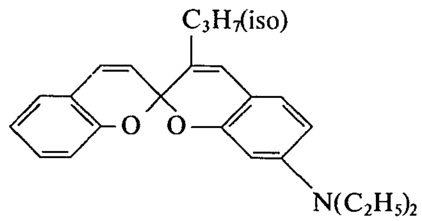

Die Verbindung schmilzt bei 140 bis 142° C.

Wird eine Lösung dieser Verbindung in Mikrokapseln eingeschlossen und auf Papier als Beschichtung aufgebracht, so erhält man beim Auflegen und Beschriften auf einer sauren Nehmerschicht, wobei die Kapseln zerstört werden und deren Inhalt mit der Nehmerschicht in Berührung gebracht wird, eine blaugraue Durchschrift.

Aufgrund der sehr geringen Selbstfarbentwicklungsfähigkeit des Farbbildners erfolgt praktisch keine Farbentwicklung (Spiegelschrift) auf dem die Kapselschicht tragenden Blatt durch die aus den zerstörten Kapseln freigesetzte Farbbildnerlösung.

Diese geringe Farbbildungstendenz zeigt der Farbbildner auch beim Durchschreiben auf nicht beschichtetem Papier, wo praktisch keine Farbentwicklung erfolgt. Demgegenüber gibt der isomere Farbbildner mit einer Isopropylgruppe in 3′-Stellung eine deutlich sichtbare blaue Durchschrift.

Beispiel 2

In die Lösung von 10 Teilen Isobutyl-(2-oxy-styryl)-keton und 16 Teilen 4-Dibenzylaminosalicylaldehyd in 120 Teilen Methanol wird bei 40 bis 50°C bis zur Sättigung Chlorwasserstoff eingeleitet. Man rührt 3 Stunden bei Raumtemperatur nach, isoliert aus dem abgekühlten Reaktionsgemisch den kristallinen Farbstoff und führt die Cyclisierung zum Farbbildner wie in Beispiel 1 beschrieben durch. Man erhält 15 Teile 3-Isopropyl-7-dibenzylamino-2,2′-spirodi-(2H-1-benzopyran) der Formel

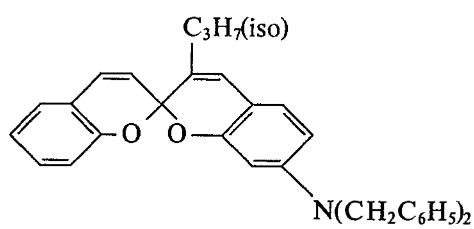

mit einem Schmelzpunkt von 119 bis 121° C.

In Kontakt mit sauer reagierenden Substanzen entwickelt die Verbindung eine Blaugraufärbung.

Beispiel 3

19 Teile n-Propyl-(2-oxy-styryl)-keton und 19 Teile 4-N-Pyrrolidinylsalicylaldehyd werden analog den Angaben in Beispiel 1 zum Farbbildner der Formel

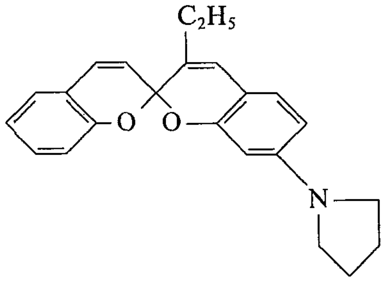

umgesetzt. Die Ausbeute an 3-Äthyl-7-N-pyrrolidinyl-2,2'-spirodi-(2H-1-benzopyran) beträgt 9 Teile, der Schmelzpunkt der Verbindung liegt bei 154 bis 156° C.

Die Substanz entwickelt mit Elektronenakzeptorsubstanzen eine Blaugraufärbung.

## Beispiel 4

9 Teile Äthyl-(2-oxy-styryl)-keton und 9,5 Teile 4-N-Pyrrolidinylsalicylaldehyd werden entsprechend den Angaben in Beispiel 1 umgesetzt. Nach dem Aufarbeiten erhält man 5 Teile 3-Methyl-7-N-pyrrolidinyl-2,2'-spirodi-(2H-1-benzopyran) der Formel

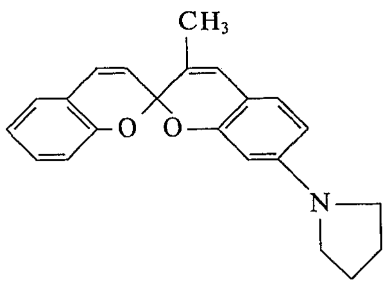

Die Verbindung schmilzt bei 150 bis 152° C und gibt in Kontakt mit sauer reagierenden Substanzen eine Blaugraufärbung.

## Beispiel 5

23 Teile 2-Methyl-3-phenyl-7-dimethylamino-benzopyrrylium-tetrachloroferrat und 8 Teile 4-Methoxy-salicylaldehyd werden in 100 Teilen Alkohol 3 1/2 Stunden unter Rückfluß erhitzt. Man rührt 12 Stunden bei Raumtemperatur nach, isoliert den kristallinen Farbstoff und führt die Cyclisierung wie in Beispiel 1 beschrieben durch. Die Ausbeute an 3-Phenyl-7-dimethylamino-7'-methoxy-2,2'-spirodi-(2H-1-benzopyran) der Formel

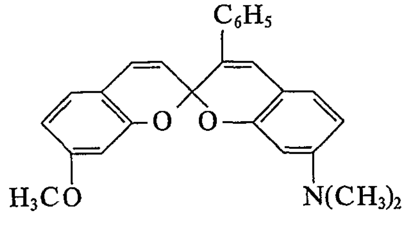

beträgt 11 Teile, der Schmelzpunkt liegt bei 148 bis 150° C.

In Kontakt mit sauer reagierenden Substanzen erhält man Grünfärbung.

Beispiele 6 bis 34

Analog den Angaben in Beispiel 1 werden Farbbildner der Formel

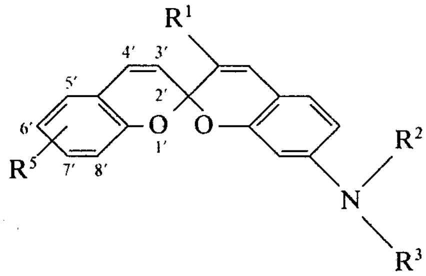

erhalten. Die Bedeutung der Substituenten $R^1$, $R^2$, $R^3$ und $R^5$ ist in der folgenden Tabelle angegeben:

| Bsp. | $R^1$ | $R^2$ | $R^3$ | $R^5$ | Farbton |
|---|---|---|---|---|---|
| 6 | $CH_3$ | $CH_3$ | $CH_3$ | 6'-Cl | blaugrau |
| 7 | $i\text{-}C_3H_7$ | $CH_3$ | $CH_3$ | 6'-Br | blaugrau |
| 8 | $C_6H_5$ | $CH_3$ | $CH_3$ | 8'-$CO_2CH_3$ | blauviolett |
| 9 | $C_6H_5$ | $CH_3$ | $CH_3$ | 8'-$CO_2C_2H_5$ | blauviolett |
| 10 | $C_6H_5$ | $CH_3$ | $CH_3$ | 6'-$C_4H_9$(tert.) | blau |
| 11 | $C_6H_5$ | $CH_3$ | $CH_3$ | 6'-$NO_2$ | blauviolett |
| 12 | $C_6H_5$ | $CH_3$ | $CH_3$ | 6'-$CH_3$ | blau |
| 13 | $C_6H_5$ | $-(CH_2)_4-$ | | 8'-$OCH_3$ | blau |
| 14 | $C_6H_5$ | $-(CH_2)_4-$ | | 7'-$OCH_3$ | blaugrün |
| 15 | $i\text{-}C_4H_9$ | $CH_3$ | $CH_3$ | H | blaugrau |
| 16 | $i\text{-}C_5H_{11}$ | $CH_3$ | $CH_3$ | H | blaugrau |
| 17 | $C_{16}H_{33}$ | $CH_3$ | $CH_3$ | H | blaugrau |
| 18 | $p\text{-}C_6H_4\text{-}CH_3$ | $CH_3$ | $CH_3$ | H | blau |
| 19 | $p\text{-}C_6H_4\text{-}Cl$ | $CH_3$ | $CH_3$ | H | blau |
| 20 | $p\text{-}C_6H_4OCH_3$ | $CH_3$ | $CH_3$ | H | blau |
| 21 | $CH_2C_6H_5$ | $CH_3$ | $CH_3$ | H | blaugrau |
| 22 | $C_2H_4C_6H_5$ | $CH_3$ | $CH_3$ | H | blaugrau |
| 23 | $i\text{-}C_3H_7$ | $C_3H_7$ | $C_3H_7$ | H | blaugrau |
| 24 | $CH_3$ | $C_{12}H_{25}$ | $C_{12}H_{25}$ | H | blaugrau |
| 25 | $CH_3$ | $CH_3$ | $p\text{-}C_6H_4Cl$ | H | blaugrau |
| 26 | $CH_3$ | $CH_3$ | $p\text{-}C_6H_4CH_3$ | H | blaugrau |
| 27 | $CH_3$ | $C_2H_4C_6H_5$ | $C_2H_4C_6H_5$ | H | blaugrau |
| 28 | $CH_3$ | $-(CH_2)_5-$ | | H | blaugrau |
| 29 | $CH_3$ | $-(CH_2)_2O(CH_2)_2-$ | | H | blaugrau |
| 30 | $CH_3$ | $-(CH_2)_2N(CH_2)_2-$<br>$CH_3$ | | H | blaugrau |
| 31 | $CH_3$ | 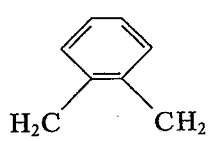 | | H | blaugrau |
| 32 | $CH_3$ | $C_2H_4CN$ | $CH_2C_6H_5$ | H | blaugrau |
| 33 | $CH_3$ | $C_2H_4CN$ | $C_2H_4CN$ | H | blaugrau |
| 34 | $CH_3$ | $C_2H_4Cl$ | $C_2H_4Cl$ | H | blaugrau |
| 35 | $CH_3$ | $C_2H_4OCH_3$ | $CH_3$ | H | blaugrau |

Beispiel 36

Die Herstellung erfolgt nach den Angaben des Beispiels 1

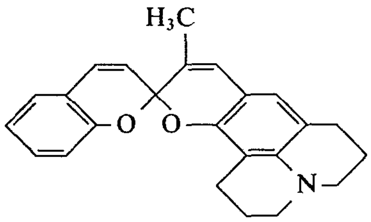

Farbton: blaugrau

Beispiel 37

Die Herstellung erfolgt nach den Angaben des Beispiels 1

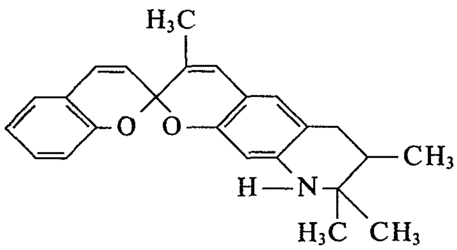

Farbton: blaugrau

Beispiel 38

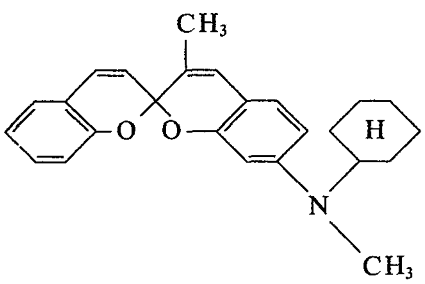

Farbton: blaugrau

Beispiel 39

46 Teile 2-Methyl-3-phenyl-7-dimethylamino-benzopyrylium-tetrachloroferrat und 17 Teile 2-Oxy-1-naphthaldehyd werden in 260 Teilen Methanol 2 Stunden unter Rückfluß erhitzt. Der kristalline Farbstoff wird isoliert und wie in Beispiel 1 beschrieben zum Farbbildner cyclisiert. Man erhält 18 Teile 3-Phenyl-7-dimethylamino-spiro-(2H-1-benzopyran)-2,2'-2H-naphtho-(2,1-b)-pyran der Formel

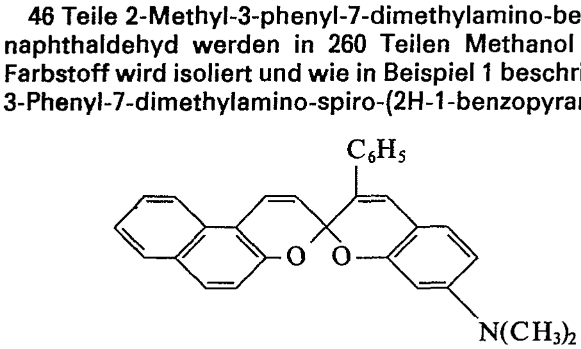

Der Farbbildner schmilzt bei 228 bis 230°C und entwickelt mit Elektronenakzeptorsubstanzen eine blaugrüne Färbung.

Beispiele 40 bis 43

Entsprechend den Angaben im Beispiel 38 werden Farbbildner der Formel

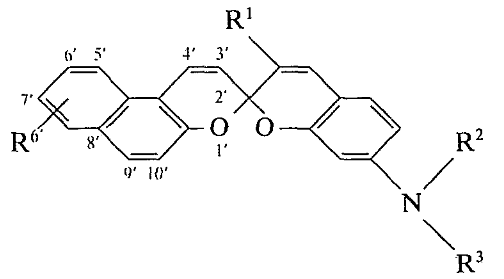

erhalten. Die Substituenten $R^1$, $R^2$, $R^3$ und $R^6$ sind in der folgenden Tabelle angegeben.

| Bsp. | $R^1$ | $R^2$ | $R^3$ | $R^6$ | Farbton |
|---|---|---|---|---|---|
| 40 | $C_6H_5$ | $CH_3$ | $CH_3$ | 7'-Cl | blau |
| 41 | $C_6H_5$ | $-(CH_2)_4-$ | | 7'-Br | blau |
| 42 | $C_6H_5$ | $CH_3$ | $CH_3$ | 10'-$CO_2CH_3$ | blau |
| 43 | $C_6H_5$ | $CH_3$ | $CH_3$ | 10'-$CO_2C_2H_5$ | blau |

**Patentansprüche**

1. Verbindungen der allgemeinen Formel

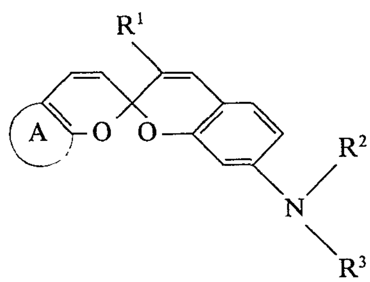

in der

A den Rest eines ankondensierten Benzolringes, der gegebenenfalls durch $C_1$- bis $C_4$-Alkyl, $C_1$- bis $C_4$-Alkoxy, Nitro, Chlor, Brom oder $C_1$- bis $C_6$-Alkoxycarbonyl substituiert ist, oder eines in 2,1-Stellung ankondensierten Naphthalinringes, der gegebenenfalls durch Chlor, Brom oder $C_1$- bis $C_6$-Alkoxycarbonyl substituiert ist,

$R^1$ $C_1$- bis $C_{16}$-Alkyl, Phenyl, durch $C_1$- bis $C_4$-Alkyl, $C_1$- bis $C_4$-Alkoxy, Chlor oder Brom substituiertes Phenyl, oder $C_7$- bis $C_{10}$-Phenalkyl und

$R^2$ und $R^3$ unabhängig voneinander Wasserstoff, $C_1$- bis $C_{12}$-Alkyl, durch Cyan, Chlor, Methoxy oder Äthoxy substituiertes $C_2$- bis $C_4$-Alkyl, $C_5$- bis $C_7$-Cycloalkyl, $C_7$- bis $C_{10}$-Phenalkyl oder ein gegebenenfalls durch 1 bis 3 Methyl substituiertes und an das in 6- und/oder 8-Stellung befindliche C-Atom des Benzringes gebundenes Trimethylen, oder

$R^2$ $C_1$- bis $C_4$-Alkyl und

$R^3$ Phenyl oder durch Chlor, Brom, $C_1$- bis $C_3$-Alkyl, Methoxy oder Äthoxy substituiertes Phenyl oder

$R^2$ Benzyl und

$R^3$ $\beta$-Cyanäthyl oder die Gruppe

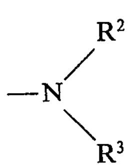

Morpholinyl, das gegebenenfalls durch 1 oder 2 Methyl substituiert ist, Pyrolidinyl, N'-$C_1$- bis -$C_4$-Alkylpiperazinyl oder Isoindolinyl bedeuten.

2. Verbindungen der Formel gemäß Anspruch 1, in der $R^1$ für $C_1$- bis $C_4$-Alkyl und A für den Rest eines ankondensierten Benzolringes stehen und $R^2$ und $R^3$ die im Anspruch 1 angegebene Bedeutung haben.

3. Verbindungen der Formel gemäß Anspruch 1, in der $R^1$ für $C_1$- bis $C_4$-Alkyl und

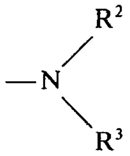 für $—N(C_2H_5)_2$,

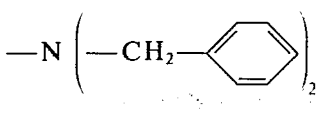 oder 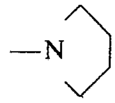

und A für den Rest eines ankondensierten Benzolringes stehen.

4. Verwendung der Verbindungen gemäß den Ansprüchen 1 bis 3 als Farbbildner für druckempfindliche Kopiersysteme.

5. Druckempfindliches Aufzeichnungsmaterial, dadurch gekennzeichnet, daß dieses als Farbbildner Verbindungen der Ansprüche 1, 2 oder 3 enthält.

**Claims**

1. A spirodipyran of the general formula

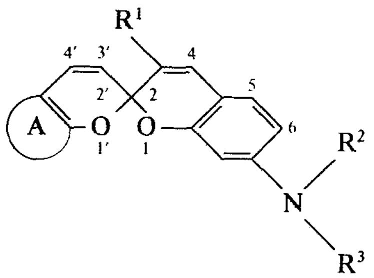

where

A is a radical of a fused benzene ring, which is unsubstituted or substituted by $C_1-C_4$-alkyl, $C_1-C_4$-alkoxy, nitro, chlorine, bromine or $C_1-C_6$-alkoxycarbonyl, or the radical of a 2,1-fused naphthalene ring which is unsubstituted or substituted by chlorine, bromine or $C_1-C_6$-alkoxycarbonyl,

$R^1$ is $C_1-C_{16}$-alkyl, phenyl which is unsubstituted or substituted by $C_1-C_4$-alkyl, $C_1-C_4$-alkoxy, chlorine or bromine, or $C_7-C_{10}$-phenylalkyl, and

$R^2$ and $R^3$ independently of one another are hydrogen, $C_1-C_{12}$-alkyl, $C_2-C_4$-alkyl which is substituted by cyano, chlorine, methoxy or ethoxy, $C_5-C_7$-cycloalkyl, $C_7-C_{10}$-phenylalkyl or trimethylene which is unsubstituted or substituted by 1, 2 or 3 methyl groups and is bonded to the carbon in the 6- and/or 8-position of the benzene ring or

$R^2$ is $C_1-C_4$-alkyl, and

$R^3$ is phenyl which is unsubstituted or substituted by chlorine, bromine, $C_1-C_3$-alkyl, methoxy or ethoxy, or

$R^2$ is benzyl, and

$R^3$ is $\beta$-cyanoethyl, or the group

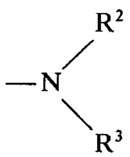

is morpholinyl which is unsubstituted or substituted by 1 or 2 methyl groups, or is pyrrolidinyl, N'-$C_1-C_4$-alkylpiperazinyl or isoindolinyl.

2. A compound of the formula as claimed in claim 1, where $R^1$ is $C_1-C_4$-alkyl, A is a radical of a fused benzene ring, and $R^2$ and $R^3$ have the meanings given in claim 1.

3. A compound of the formula as claimed in claim 1, where $R^1$ is $C_1-C_4$-alkyl,

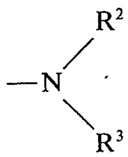

is $-N(C_2H_5)_2$,

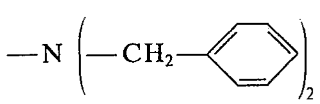

or

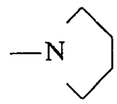

and A is a radical of a fused benzene ring.

4. Use of a compound as claimed in claims 1 to 3 as dyeforming component for pressure-sensitive copying systems.

5. A pressure-sensitive recording material, characterized in that it contains, as dye-forming component, a compound of claim 1, 2 or 3.

**Revendications**

1. Spirodipyrannes de formule générale

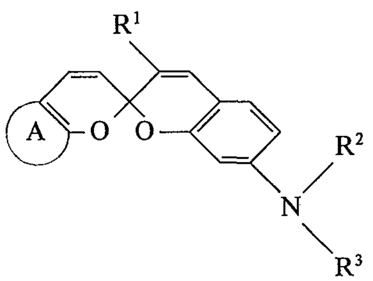

dans laquelle

A représente le reste d'un cycle benzénique condensé et éventuellement substitué par des groupes alkyle en C1 – C4, alcoxy en C1 – C4, nitro, des atomes de chlore, de brome ou des groupes (alcoxy en C1 – C6)-carbonyle, ou un cycle naphtalénique condensé en position 2,1 et éventuellement substitué par le chlore, le brome ou un groupe (alcoxy en C1 – C6)-carbonyle,

$R^1$ représente un groupe alkyle en C1 – C16, phényle, phényle substitué par des groupes alkyle en C1 – C4, alcoxy en C1 – C4, le chlore ou le brome, ou phénylalkyle en C7 – C10, et

$R^2$ et $R^3$ représentent chacun, indépendamment l'un de l'autre, l'hydrogène, un groupe alkyle en C1 – C12, un groupe alkyle en C2 – C4 portant des substituants cyano, chloro, méthoxy ou éthoxy, un groupe cycloalkyle en C5 – C7, un groupe phénylalkyle en C7 – C10 ou un groupe triméthylène portant éventuellement 1 à 3 substituants méthyle et fixé sur l'atome de carbone en position 6 et/ou 8 du cycle benzénique, ou bien

$R^2$ représente un groupe alkyle en C1 – C4 et

$R^3$ représente un groupe phényle ou phényle substitué par le chlore, le brome, des groupes alkyle en C1 – C3, méthoxy ou éthoxy, ou bien

$R^2$ représente le groupe benzyle et

$R^3$ le groupe bêta-cyanéthyle ou le groupe

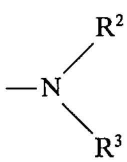

morpholinyle éventuellement substitué par 1 ou 2 groupes méthyle, pyrrolidinyle, N'-(alkyle en C1 – C4)-pipérazinyle ou isoindolynyle.

2. Composés répondant à la formule de la revendication 1 dans laquelle $R^1$ représente un groupe alkyle en C1 – C4 et A le reste d'un cycle benzénique condensé, $R^2$ et $R^3$ ayant les significations indiquées dans la revendication 1.

3. Composés répondant à la formule de la revendication 1, dans laquelle $R^1$ représente un groupe alkyle en C1 – C4 et

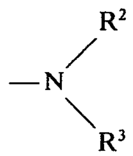 représente $—N(C_2H_5)_2$,

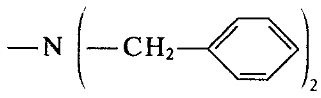 ou 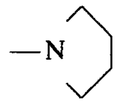

et A le reste d'un cycle benzénique condensé.

4. Utilisation des composés selon les revendications 1 à 3 en tant que que chromogènes pour des systèmes à copier sensibles à la pression.

5. Matières à tracer sensibles à la pression, caractérisée en ce qu'elle contient en tant que chromogènes des composés des revendications 1, 2 ou 3.